# EUROPEAN PATENT APPLICATION

(11) **EP 4 678 107 A1**
(43) Date of publication of application: **14.01.2026**
(21) Application number: 25187014.3
(22) Date of filing: 02.07.2025
(51) Int. Cl.: A61B 6/02, A61B 6/00, A61B 6/50

(54) **IMAGE PROCESSING APPARATUS, IMAGE PROCESSING METHOD, AND IMAGE PROCESSING PROGRAM**

(30) Priority: 12.07.2024 JP 2024112902
(71) Applicant: FUJIFILM Corporation, Tokyo 106-8620 (JP)
(72) Inventor: FUKUDA, Wataru, Kanagawa, 258-8538 (JP)
(74) Representative: Dehns Germany Partnerschaft mbB

(57) **Abstract**

An image processing apparatus (16) includes a CPU. The CPU acquires a series of a plurality of projection images or a plurality of tomographic images obtained by performing tomosynthesis imaging by irradiating a breast with radiation having a first energy; acquires a plurality of normal two-dimensional images captured by irradiating the breast with radiation having a second energy, which is higher than the first energy, a plurality of times; calculates, for each of the plurality of normal two-dimensional images, a virtual projection position, which is a position at which the breast is virtually projected during the tomosynthesis imaging, from a position of a radiation source in a case in which the normal two-dimensional image is captured; generates a composite two-dimensional image from the plurality of projection images or the plurality of tomographic images based on the virtual projection position calculated for each of the plurality of normal two-dimensional images; and generates a difference image between each of the plurality of normal two-dimensional images and each of the composite two-dimensional images generated for each of the plurality of normal two-dimensional images.

## Description

### BACKGROUND OF THE INVENTION

### 1. Field of the Invention

The present disclosure relates to an image processing apparatus, an image processing method, and an image processing program.

### 2. Description of the Related Art

A technology known as a contrast enhanced digital mammography (CEDM) biopsy is known, which generates a radiation image in which a contrast medium is enhanced by irradiating a breast, in which the contrast medium has been injected, with radiation having different energies to capture a low-energy image and a high-energy image and generating a difference image between the high-energy image and the low-energy image.

For example, JP7446454B discloses an information processing apparatus that captures a high-energy image a plurality of times after capturing a low-energy image, and generates a difference image.

### SUMMARY OF THE INVENTION

In the CEDM biopsy, since a set of the high-energy image and the low-energy image is captured a plurality of times while changing an angle of the radiation source, an exposure dose is increased. In particular, since the exposure dose is large in a case of capturing the low-energy image, it is desired to reduce the exposure dose.

The present disclosure has been made in view of the above-described circumstances, and an object of the present disclosure is to provide an image processing apparatus, an image processing method, and an image processing program capable of reducing an exposure dose during a CEDM biopsy.

In order to achieve the above-described object, a first aspect of the present disclosure provides an image processing apparatus comprising: a processor, in which the processor is configured to: acquire a series of a plurality of projection images or a plurality of tomographic images obtained by performing tomosynthesis imaging by irradiating a breast with radiation having a first energy; acquire a plurality of normal two-dimensional images captured by irradiating the breast with radiation having a second energy, which is higher than the first energy, a plurality of times; calculate, for each of the plurality of normal two-dimensional images, a virtual projection position, which is a position at which the breast is virtually projected during the tomosynthesis imaging, from a position of a radiation source in a case in which the normal two-dimensional image is captured; generate a composite two-dimensional image from the plurality of projection images or the plurality of tomographic images based on the virtual projection position calculated for each of the plurality of normal two-dimensional images; and generate a difference image between each of the plurality of normal two-dimensional images and each of the composite two-dimensional images generated for each of the plurality of normal two-dimensional images.

A first embodiment of the present disclosure provides the image processing apparatus according to the first aspect, in which the processor is configured to, in a case in which the plurality of projection images are acquired, generate the plurality of tomographic images from the plurality of projection images based on the virtual projection position and then generate the composite two-dimensional image from the plurality of tomographic images.

A second embodiment of the present disclosure provides the image processing apparatus according to the first embodiment, in which the processor is configured to generate the composite two-dimensional image from the plurality of tomographic images based on a projection path from the virtual projection position.

A third embodiment of the present disclosure provides the image processing apparatus according to the first embodiment, in which the processor is configured to: generate the plurality of tomographic images by correcting a magnification ratio using the virtual projection position as a center for each of the plurality of projection images; and generate the composite two-dimensional image by performing a parallel projection on the plurality of generated tomographic images.

A fourth embodiment of the present disclosure provides the image processing apparatus according to the first aspect, in which the processor is configured to, in a case in which the plurality of tomographic images are acquired, generate the composite two-dimensional image by combining the plurality of tomographic images based on the virtual projection position.

A fifth embodiment of the present disclosure provides the image processing apparatus according to the first aspect, in which the processor is configured to acquire the plurality of tomographic images of which magnification ratios are corrected using the virtual projection position as a center.

An image processing apparatus according to a sixth embodiment of the present disclosure provides the image processing apparatus according to the first aspect, in which the processor is configured to: acquire the plurality of tomographic images and correct magnification ratios of the plurality of tomographic images at the virtual projection position in a case in which the magnification ratios of the plurality of tomographic images are not corrected or centers of the correction of the magnification ratios are at different positions; and generate the composite two-dimensional image by performing a parallel projection on the plurality of tomographic images subjected to the correction.

A seventh embodiment of the present disclosure provides the image processing apparatus according to the first aspect, in which the normal two-dimensional image includes a plurality of two-dimensional images captured while changing the position of the radiation source.

An eighth embodiment of the present disclosure provides the image processing apparatus according to the first aspect, in which the processor is configured to, in a case in which movement of the breast is detected during the tomosynthesis imaging, perform control of performing the tomosynthesis imaging again.

A ninth embodiment of the present disclosure provides the image processing apparatus according to the first aspect, in which the processor is configured to, in a case in which movement of the breast is detected during the tomosynthesis imaging, generate the composite two-dimensional image by correcting the movement of the breast.

A tenth embodiment of the present disclosure provides the image processing apparatus according to the eighth or ninth embodiments, in which the processor is configured to detect the movement of the breast by performing threshold value processing on a difference between the normal two-dimensional image and the composite two-dimensional image.

An eleventh embodiment of the present disclosure provides the image processing apparatus according to the first aspect, in which the processor is configured to, in a case in which an instruction from a user is received after the tomosynthesis imaging, perform control of performing the tomosynthesis imaging again.

A second aspect of the present disclosure provides an image processing apparatus comprising: a processor, in which the processor is configured to: acquire a series of a plurality of projection images or a plurality of tomographic images obtained by performing tomosynthesis imaging by irradiating a breast with radiation having a first energy; acquire a plurality of normal two-dimensional images captured by irradiating the breast with radiation having a second energy lower than the first energy a plurality of times; calculate, for each of the plurality of normal two-dimensional images, a virtual projection position, which is a position at which the breast is virtually projected during the tomosynthesis imaging, from a position of a radiation source in a case in which the normal two-dimensional image is captured; generate a composite two-dimensional image from the plurality of projection images or the plurality of tomographic images based on the virtual projection position calculated for each of the plurality of normal two-dimensional images; and generate a difference image between each of the plurality of normal two-dimensional images and each of the composite two-dimensional images generated for each of the plurality of normal two-dimensional images.

A third aspect of the present disclosure provides an image processing method executed by a computer, the image processing method comprising: acquiring a series of a plurality of projection images or a plurality of tomographic images obtained by performing tomosynthesis imaging by irradiating a breast with radiation having a first energy; acquiring a plurality of normal two-dimensional images captured by irradiating the breast with radiation having a second energy, which is higher than the first energy, a plurality of times; calculating, for each of the plurality of normal two-dimensional images, a virtual projection position, which is a position at which the breast is virtually projected during the tomosynthesis imaging, from a position of a radiation source in a case in which the normal two-dimensional image is captured; generating a composite two-dimensional image from the plurality of projection images or the plurality of tomographic images based on the virtual projection position calculated for each of the plurality of normal two-dimensional images; and generating a difference image between each of the plurality of normal two-dimensional images and each of the composite two-dimensional images generated for each of the plurality of normal two-dimensional images.

A fourth aspect of the present disclosure provides an image processing program causing a computer to execute a process comprising: acquiring a series of a plurality of projection images or a plurality of tomographic images obtained by performing tomosynthesis imaging by irradiating a breast with radiation having a first energy; acquiring a plurality of normal two-dimensional images captured by irradiating the breast with radiation having a second energy, which is higher than the first energy, a plurality of times; calculating, for each of the plurality of normal two-dimensional images, a virtual projection position, which is a position at which the breast is virtually projected during the tomosynthesis imaging, from a position of a radiation source in a case in which the normal two-dimensional image is captured; generating a composite two-dimensional image from the plurality of projection images or the plurality of tomographic images based on the virtual projection position calculated for each of the plurality of normal two-dimensional images; and generating a difference image between each of the plurality of normal two-dimensional images and each of the composite two-dimensional images generated for each of the plurality of normal two-dimensional images.

According to the present disclosure, it is possible to reduce the exposure dose during the CEDM biopsy.

### BRIEF DESCRIPTION OF THE DRAWINGS

Fig. 1 is a configuration diagram schematically showing an example of an overall configuration of a radiographic imaging system according to an embodiment.
Fig. 2 is a diagram showing an example of tomosynthesis imaging.
Fig. 3 is a block diagram showing an example of a configuration of an image processing apparatus according to the embodiment.
Fig. 4 is a diagram showing a CEDM biopsy according to a comparative example.
Fig. 5 is a diagram showing a processing flow of the CEDM biopsy according to the comparative example.
Fig. 6 is a block diagram showing an example of a functional configuration of an image processing apparatus according to a first embodiment.
Fig. 7A is a diagram showing an example of a case in which a high-energy normal two-dimensional image is captured from an irradiation position of a radiation source.
Fig. 7B is a diagram showing processing of calculating a virtual projection position corresponding to the irradiation position of the radiation source during the tomosynthesis imaging.
Fig. 8 is a diagram showing a magnification ratio.
Fig. 9 is a diagram showing a processing flow of a CEDM biopsy according to the first embodiment.
Fig. 10 is a diagram showing the CEDM biopsy according to the first embodiment.
Fig. 11 is a flowchart showing an example of a flow of processing by an image processing program according to the first embodiment.
Fig. 12 is a flowchart showing another example of the flow of the processing by the image processing program according to the first embodiment.
Fig. 13 is a diagram showing a processing flow of a CEDM biopsy according to a second embodiment.
Fig. 14 is a diagram showing the CEDM biopsy according to the second embodiment.

### DESCRIPTION OF THE PREFERRED EMBODIMENTS

Hereinafter, embodiments of the present disclosure will be described in detail with reference to the drawings. Each embodiment does not limit the present disclosure.

### First Embodiment

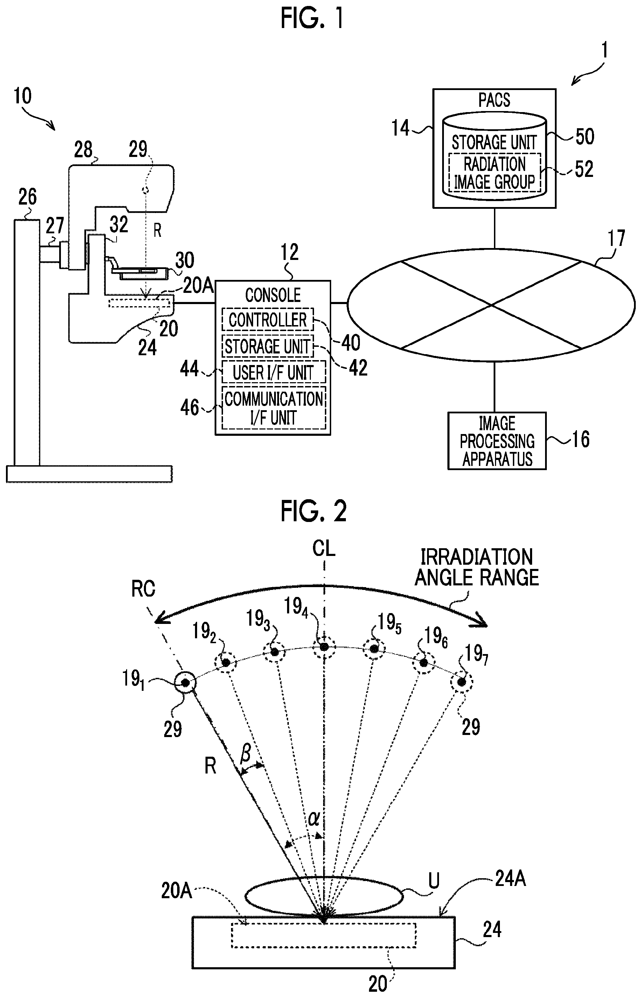

First, an example of an overall configuration of a radiographic imaging system according to the present embodiment will be described. Fig. 1 is a configuration diagram showing an example of an overall configuration of a radiographic imaging system 1 according to the present embodiment. As shown in Fig. 1, a radiographic imaging system 1 according to the present embodiment comprises a mammography apparatus 10, a console 12, a picture archiving and communication systems (PACS) 14, and an image processing apparatus 16. The console 12, the PACS 14, and the image processing apparatus 16 are connected via a network 17 by wired communication or wireless communication.

First, the mammography apparatus 10 according to the present embodiment will be described. In Fig. 1, a side view showing an example of an appearance of the mammography apparatus 10 according to the present embodiment is shown. It should be noted that Fig. 1 shows an example of the appearance in a case in which the mammography apparatus 10 is viewed from a left side of a person under examination.

The mammography apparatus 10 according to the present embodiment is an apparatus that is operated under the control of the console 12, and is configured to capture, using a breast of the person under examination as a subject, a radiation image of a breast by irradiating the breast with radiation R (for example, X-rays) emitted from a radiation source 29. It should be noted that the radiation source 29 is, for example, a tube that emits the radiation R. Further, the mammography apparatus 10 according to the present embodiment has a function of performing normal imaging for capturing images by arranging the radiation source 29 at an irradiation position along a normal direction to a detection surface 20A of a radiation detector 20 and so-called tomosynthesis imaging (which will be described below) for capturing images by moving the radiation source 29 to each of a plurality of irradiation positions. The tomosynthesis imaging is a function of generating a radiation image corresponding to a normal two-dimensional image obtained by normal imaging by combining a series of a plurality of projection images obtained by irradiating the breast with radiation or a plurality of tomographic images generated from the series of projection images.

As shown in Fig. 1, the mammography apparatus 10 comprises an imaging table 24, a base 26, an arm portion 28, and a compression unit 32.

A radiation detector 20 is disposed inside the imaging table 24. As shown in Fig. 2, in the mammography apparatus 10 according to the present embodiment, in a case in which the imaging is performed, a breast U of the person under examination is positioned on an imaging surface 24A of the imaging table 24 by a user.

The radiation detector 20 detects the radiation R that has been transmitted through the breast U as the subject. Specifically, the radiation detector 20 detects the radiation R that enters the breast U of the person under examination and the imaging table 24 and that reaches the detection surface 20A of the radiation detector 20, generates a radiation image based on the detected radiation R, and outputs image data representing the generated radiation image. Hereinafter, the series of operations of irradiating the breast with the radiation R emitted from the radiation source 29 to generate the radiation image via the radiation detector 20 may be referred to as "imaging". A type of the radiation detector 20 according to the present embodiment is not particularly limited, and for example, the radiation detector 20 may be an indirect conversion type radiation detector that converts the radiation R into light and converts the converted light into charge, or may be a direct conversion type radiation detector that directly converts the radiation R into charge.

The compression plate 30 used to compress the breast in a case of performing imaging is attached to the compression unit 32 provided on the imaging table 24 and is moved in a direction approaching or departing from the imaging table 24 (hereinafter, referred to as an "up-down direction") by a compression plate drive unit (not shown) provided in the compression unit 32. The compression plate 30 is moved in the up-down direction to compress the breast of the person under examination between the imaging table 24 and the compression plate 30.

The arm portion 28 can rotate relative to the base 26 via a shaft portion 27. The shaft portion 27 is fixed to the base 26, and the shaft portion 27 and the arm portion 28 rotate as one body. Gears are provided in each of the shaft portion 27 and the compression unit 32 of the imaging table 24, and the gears are switched between an engaged state and a non-engaged state, so that a state in which the compression unit 32 of the imaging table 24 and the shaft portion 27 are connected to each other and are rotated integrally and a state in which the shaft portion 27 is separated from the imaging table 24 and rotates freely can be switched. It should be noted that the elements for switching between transmission and non-transmission of power of the shaft portion 27 are not limited to the gears, and various mechanical elements can be used. The arm portion 28 and the imaging table 24 can be separately rotated relative to the base 26 with the shaft portion 27 as a rotation axis.

In a case in which the tomosynthesis imaging is performed in the mammography apparatus 10, the radiation source 29 is sequentially moved to each of the plurality of irradiation positions having different irradiation angles in accordance with the rotation of the arm portion 28. The radiation source 29 has a radiation tube (not shown) that generates the radiation R, and the radiation tube is moved to each of the plurality of irradiation positions in accordance with the movement of the radiation source 29. Fig. 2 is a diagram showing an example of the tomosynthesis imaging. It should be noted that, in Fig. 2, the compression plate 30 is not shown. In the present embodiment, as shown in Fig. 2, the radiation source 29 is moved to irradiation positions 19ₜ (t = 1, 2, ...; the maximum value is 7 in Fig. 2) having different irradiation angles at an interval of a predetermined angle β, that is, positions at which the irradiation angle of the radiation R with respect to the detection surface 20A of the radiation detector 20 are different from each other. At each irradiation position 19ₜ, the breast U is irradiated with the radiation R emitted from the radiation source 29 in accordance with an instruction of the console 12, and the radiation image is captured by the radiation detector 20. In the radiographic imaging system 1, in a case in which the tomosynthesis imaging is performed by moving the radiation source 29 to each irradiation position 19ₜ to capture the radiation image at each irradiation position 19ₜ, seven radiation images are obtained in the example of Fig. 2.

It should be noted that, during the tomosynthesis imaging, in a case in which the radiation image captured at each irradiation position 19 is described separately from other radiation images, the radiation image will be referred to as a "projection image", and a plurality of projection images captured in one tomosynthesis imaging will be referred to as a "series of a plurality of projection images".

It should be noted that, as shown in Fig. 2, the irradiation angle of the radiation R refers to an angle α formed between a normal line CL of the detection surface 20A of the radiation detector 20 and a radiation axis RC. The radiation axis RC refers to an axis that connects a focus of the radiation source 29 at each irradiation position 19 and a preset position, such as a center of the detection surface 20A. Further, here, it is assumed that the detection surface 20A of the radiation detector 20 is substantially parallel to the imaging surface 24A.

Meanwhile, in a case in which the mammography apparatus 10 performs the normal imaging, the radiation source 29 remains at the irradiation position 19ₜ (the irradiation position 19ₜ along the normal direction, the irradiation position 19₄ in Fig. 2) at which the irradiation angle α is 0 degrees. The radiation R is emitted from the radiation source 29 in accordance with the instruction of the console 12, and the radiation image is captured by the radiation detector 20. In the present embodiment, the radiation image captured during the normal imaging will be referred to as a "normal two-dimensional image" in a case in which the radiation image is described as being distinguished from other radiation images.

The mammography apparatus 10 and the console 12 are connected by wired communication or wireless communication. The radiation image captured by the radiation detector 20 in the mammography apparatus 10 is output to the console 12 by wired communication or wireless communication via a communication interface (I/F) unit (not shown).

As shown in Fig. 1, the console 12 according to the present embodiment comprises a controller 40, a storage unit 42, a user I/F unit 44, and a communication I/F unit 46.

As described above, the controller 40 of the console 12 has a function of controlling the capturing of the radiation image of the breast via the mammography apparatus 10. Examples of the controller 40 include a computer system comprising a central processing unit (CPU), a read only memory (ROM), and a random access memory (RAM).

The storage unit 42 has a function of storing, for example, information on the capturing of the radiation image, or the radiation image acquired from the mammography apparatus 10. The storage unit 42 is a non-volatile storage unit, and is, as an example, a hard disk drive (HDD) and a solid state drive (SSD).

The user I/F unit 44 includes input devices, such as various buttons and switches operated by the user such as a technician, regarding the capturing of the radiation image, and display devices, such as a lamp and a display, for displaying information on the imaging or the radiation image.

The communication I/F unit 46 performs communication of various types of data, such as information on the capturing of the radiation image or the radiation image obtained by the imaging, to and from the mammography apparatus 10 by wired communication or wireless communication. In addition, the communication I/F unit 46 performs communication of various types of data, such as the radiation image, with the PACS 14 and the image processing apparatus 16 via the network 17 by wired communication or wireless communication.

In addition, as shown in Fig. 1, the PACS 14 according to the present embodiment comprises a storage unit 50 that stores a radiation image group 52 and a communication I/F unit (not shown). The radiation image group 52 includes radiation images that are captured by the mammography apparatus 10 and that are acquired from the console 12 via a communication I/F unit (not shown) and the like.

The image processing apparatus 16 is used by a doctor or the like (hereinafter, simply referred to as "doctor") to interpret the radiation image. The image processing apparatus 16 according to the present embodiment has a function of generating a composite two-dimensional image from a series of a plurality of projection images or a plurality of tomographic images. The plurality of tomographic images can be obtained from the series of the plurality of projection images. The plurality of tomographic images are generated, for example, by reconstructing the series of the plurality of projection images using a simple back projection method, a filtered back projection method, a sequential reconstruction method, or the like. The "composite two-dimensional image" is a pseudo two-dimensional image generated by combining the plurality of tomographic images. The composite two-dimensional image is generated by combining the plurality of tomographic images having different distances (positions in a height direction) from the detection surface 20A of the radiation detector 20 to the radiation source 29 side, for example, by an addition method, an averaging method, a maximum value projection method, a minimum value projection method, or the like.

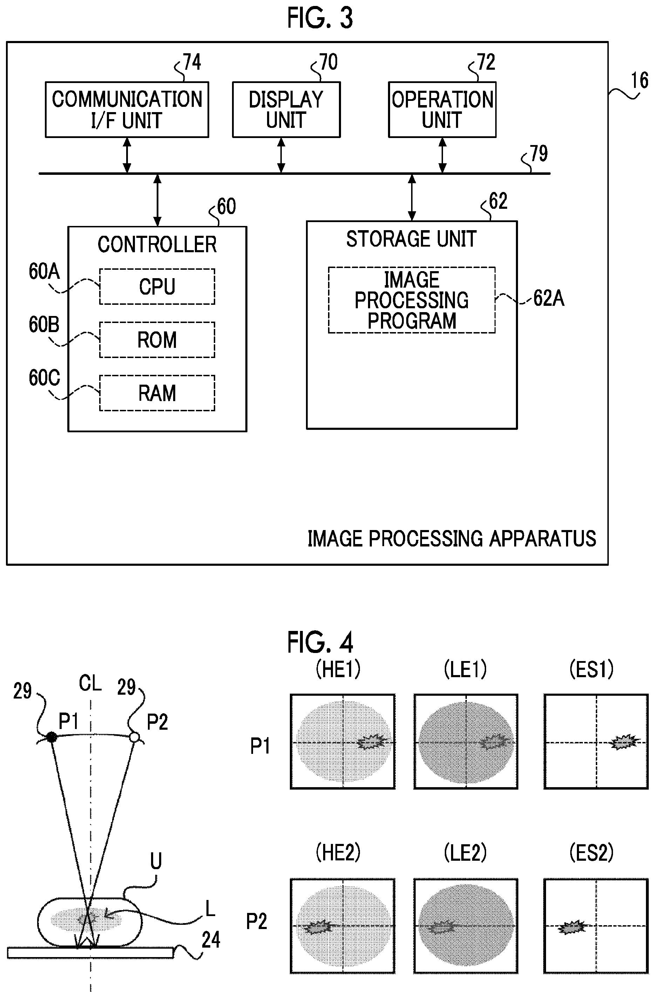

Fig. 3 is a block diagram showing an example of a configuration of the image processing apparatus 16 according to the present embodiment. As shown in Fig. 3, the image processing apparatus 16 according to the present embodiment comprises a controller 60, a storage unit 62, a display unit 70, an operation unit 72, and a communication I/F unit 74. The controller 60, the storage unit 62, the display unit 70, the operation unit 72, and the communication I/F unit 74 are connected to each other via a bus 79, such as a system bus or a control bus, such that various types of information can be transmitted and received.

The controller 60 controls the overall operation of the image processing apparatus 16. The controller 60 comprises a CPU 60A, a ROM 60B, and a RAM 60C. Various programs and the like used by the CPU 60A for the control are stored in the ROM 60B in advance. The RAM 60C temporarily stores various types of data.

The storage unit 62 is a non-volatile storage unit and is, as a specific example, an HDD or an SSD. The storage unit 62 stores an image processing program 62A according to the present embodiment.

The display unit 70 displays the radiation images or various types of information. The display unit 70 is not particularly limited, and various displays and the like may be used. In addition, the operation unit 72 is used by the doctor to input instructions for a diagnosis for a lesion of the breast using the radiation image, the user to input various types of information, or the like. The operation unit 72 is not particularly limited, and examples of the operation unit 72 include various switches, a touch panel, a touch pen, and a mouse. It should be noted that the display unit 70 and the operation unit 72 may be integrated into a touch panel display.

The communication I/F unit 74 performs communication of various types of information between the console 12 and the PACS 14 via the network 17 by wireless communication or wired communication.

Fig. 4 is a diagram showing a CEDM biopsy according to a comparative example. In the CEDM biopsy, a normal two-dimensional image (hereinafter, referred to as a "low-energy normal two-dimensional image") captured by emitting radiation having a first energy and a normal two-dimensional image (hereinafter, referred to as a "high-energy normal two-dimensional image") captured by emitting radiation having a second energy higher than the first energy are acquired at a predetermined irradiation position of the radiation source 29, and a difference image between the low-energy normal two-dimensional image and the high-energy normal two-dimensional image is created. During the CEDM biopsy, an error occurs in a case in which the irradiation position of the radiation source 29 is shifted, and thus the high-energy normal two-dimensional image and the low-energy normal two-dimensional image are captured as a set at the same irradiation position. In the example of Fig. 4, at an irradiation position P1 of the radiation source 29, a low-energy normal two-dimensional image LE1 and a high-energy normal two-dimensional image HE1 are acquired, and a difference image ES1 between the low-energy normal two-dimensional image LE1 and the high-energy normal two-dimensional image HE1 is created. In addition, a low-energy normal two-dimensional image LE2 and a high-energy normal two-dimensional image HE2 are acquired at an irradiation position P2 of the radiation source 29 by changing the angle, and a difference image ES2 between the low-energy normal two-dimensional image LE2 and the high-energy normal two-dimensional image HE2 is created. That is, the position of the lesion L can be three-dimensionally specified by the stereo principle. It should be noted that, in a case in which it is not necessary to particularly distinguish between the high-energy normal two-dimensional image, the low-energy normal two-dimensional image, and the difference image, the high-energy normal two-dimensional image, the low-energy normal two-dimensional image, and the difference image will be referred to as a high-energy normal two-dimensional image HE, a low-energy normal two-dimensional image LE, and a difference image ES, respectively.

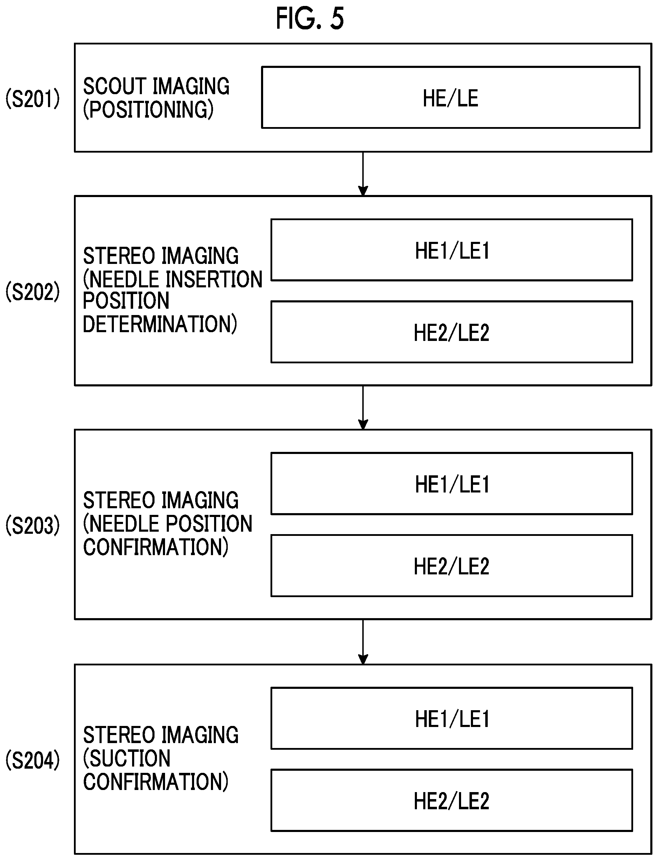

Fig. 5 is a diagram showing a processing flow of the CEDM biopsy according to the comparative example.

In (S201) of Fig. 5, scout imaging (positioning) for performing positioning is performed. During the scout imaging, the high-energy normal two-dimensional image HE and the low-energy normal two-dimensional image LE are captured.

In (S202), stereo imaging (needle insertion position determination) for determining a position of a needle to be inserted into the breast U is performed. During the stereo imaging in this case, for example, the high-energy normal two-dimensional image HE1 and the low-energy normal two-dimensional image LE1 are captured at the irradiation position P1 of the radiation source 29, and the high-energy normal two-dimensional image HE2 and the low-energy normal two-dimensional image LE2 are captured at the irradiation position P2 of the radiation source 29 by changing the angle.

In (S203), stereo imaging (needle position confirmation) for confirming a position after the needle is inserted into the breast U is performed. The stereo imaging in this case is also performed at the irradiation positions P1 and P2 in the same manner as the stereo imaging in (S202) described above.

In (S204), stereo imaging (suction confirmation) for confirming a state after suctioning a tissue from a hole of the needle is performed. The stereo imaging in this case is also performed at the irradiation positions P1 and P2 in the same manner as the stereo imaging in (S202) described above.

As described above, in the CEDM biopsy, since a set of the high-energy normal two-dimensional image HE and the low-energy normal two-dimensional image LE is captured a plurality of times while changing the angle of the radiation source 29, the exposure dose is increased. In particular, since the exposure dose is large in a case in which the low-energy normal two-dimensional image LE is captured, it is desired to reduce the exposure dose.

In the radiographic imaging system 1 according to the present embodiment, the tomosynthesis imaging is performed with radiation having low energy, and the high-energy normal two-dimensional image HE is captured. Then, the radiographic imaging system 1 generates a low-energy composite two-dimensional image virtually projected from the irradiation position of the radiation source 29 in a case in which the high-energy normal two-dimensional image HE is captured, for the plurality of projection images or the plurality of tomographic images obtained by the tomosynthesis imaging. It should be noted that, hereinafter, the low-energy composite two-dimensional image will be referred to as a low-energy composite two-dimensional image LEs in order to distinguish the low-energy composite two-dimensional image from the low-energy normal two-dimensional image LE. Then, the radiographic imaging system 1 generates the difference image ES between the high-energy normal two-dimensional image HE and the low-energy composite two-dimensional image LEs. That is, as compared with the CEDM biopsy according to the comparative example, the tomosynthesis imaging with low energy can be completed only once, and thus the exposure dose can be reduced.

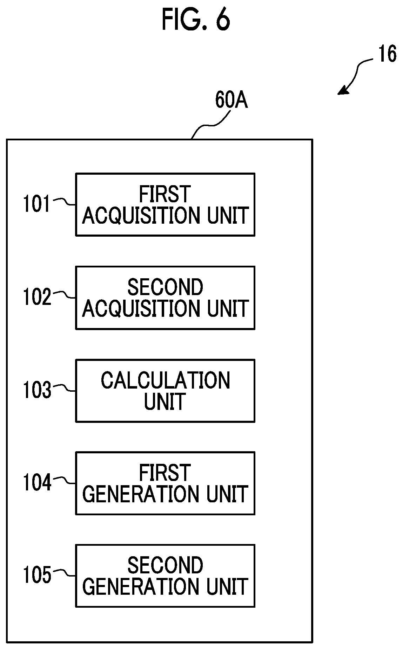

Specifically, the CPU 60A of the image processing apparatus 16 according to the present embodiment functions as each unit shown in Fig. 6 by writing the image processing program 62A stored in the storage unit 25 in the RAM 60C and executing the image processing program 62A.

Fig. 6 is a block diagram showing an example of a functional configuration of the image processing apparatus 16 according to the first embodiment. The CPU 60A of the image processing apparatus 16 according to the present embodiment functions as a first acquisition unit 101, a second acquisition unit 102, a calculation unit 103, a first generation unit 104, and a second generation unit 105. It should be noted that the first acquisition unit 101 and the second acquisition unit 102 are distinguished for convenience, but may be implemented as one acquisition unit. Similarly, the first generation unit 104 and the second generation unit 105 are distinguished for convenience, but may be implemented as one generation unit. In the present embodiment, the first energy is low energy, and the second energy is high energy.

The first acquisition unit 101 acquires the series of the plurality of projection images or the plurality of tomographic images. These plurality of projection images or plurality of tomographic images are images obtained by performing the tomosynthesis imaging by irradiating the breast U with radiation having low energy.

The second acquisition unit 102 acquires the plurality of normal two-dimensional images obtained by irradiating the breast U with radiation having high energy a plurality of times. Here, "a plurality of times" refers to four events of "positioning", "needle insertion position determination", "needle position confirmation", and "suction confirmation" in the example of Fig. 5 described above. That is, the high-energy normal two-dimensional image HE is captured four times. However, in the "needle insertion position determination", the "needle position confirmation", and the "suction confirmation", the stereo imaging is performed by changing the angle, but the stereo imaging is also counted as once in the same manner as the scout imaging.

The calculation unit 103 calculates the virtual projection position during the tomosynthesis imaging from the irradiation position of the radiation source 29 in a case in which the high-energy normal two-dimensional image HE is captured, for each of the plurality of high-energy normal two-dimensional images HE. The virtual projection position refers to a position at which the breast U is virtually projected during the tomosynthesis imaging. It should be noted that, since the "needle insertion position determination", the "needle position confirmation", and the "suction confirmation" are performed by the stereo imaging, the virtual projection positions corresponding to the respective irradiation positions of the stereo imaging may be calculated.

The first generation unit 104 generates the low-energy composite two-dimensional image LEs from the plurality of projection images or the plurality of tomographic images based on the virtual projection position calculated for each of the plurality of high-energy normal two-dimensional images HE.

The second generation unit 105 generates the difference image ES between each of the plurality of high-energy normal two-dimensional images HE and each of the low-energy composite two-dimensional images LEs generated for each of the plurality of high-energy normal two-dimensional images HE.

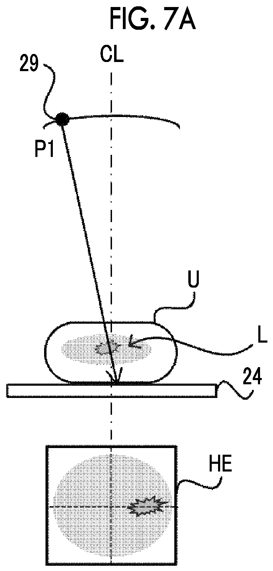

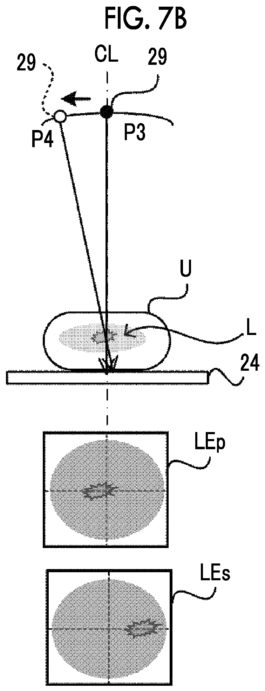

Here, virtual projection position calculation processing according to the first embodiment will be described in detail with reference to Figs. 7A and 7B.

Fig. 7A is a diagram showing an example of a case in which the high-energy normal two-dimensional image HE is captured from the irradiation position P1 of the radiation source 29. Fig. 7B is a diagram showing processing of calculating a virtual projection position P4 corresponding to the irradiation position P1 of the radiation source 29 during the tomosynthesis imaging.

As shown in Fig. 7A, the irradiation position P1 of the radiation source 29 is, for example, a position shifted to the left side from the normal line CL. The high-energy normal two-dimensional image HE is generated by performing normal imaging by irradiating the breast U with radiation having high energy from the irradiation position P1.

As shown in Fig. 7B, it is assumed that the breast U is irradiated with radiation having low energy from the irradiation position P3 of the radiation source 29 during the tomosynthesis imaging. The irradiation position P3 is one of the plurality of irradiation positions during the tomosynthesis imaging, and is a reference position for the tomosynthesis imaging. In such a case, since the irradiation position P1 and the irradiation position P3 are different positions, the position of the lesion L in the projection image LEp captured at the irradiation position P3 is shifted from the position of the lesion L in the high-energy normal two-dimensional image HE captured at the irradiation position P1. Therefore, the calculation unit 103 calculates the corresponding virtual projection position P4 from the irradiation position P1 of the high-energy normal two-dimensional image HE in order to match the positional relationship of the lesion L with the high-energy normal two-dimensional image HE. Specifically, for example, a difference between the irradiation position P1 of the high-energy normal two-dimensional image HE and the irradiation position P3 of the projection image LEp may be obtained, and the irradiation position P3 may be corrected in accordance with the obtained difference. The virtual projection position P4 is calculated for each irradiation position during the tomosynthesis imaging based on the obtained difference. That is, since the tomosynthesis imaging is continuously performed at a plurality of irradiation positions, the virtual projection positions corresponding to the respective irradiation positions may be calculated by correcting the respective irradiation positions in accordance with the difference. The first generation unit 104 generates the low-energy composite two-dimensional image LEs from the plurality of projection images or the plurality of tomographic images based on the calculated virtual projection position P4.

Next, a case will be described in which the projection image is acquired without acquiring the tomographic image and the low-energy composite two-dimensional image LEs is generated using the virtual projection position P4 as an example. The first acquisition unit 101 acquires the plurality of projection images. In a case in which the plurality of projection images are acquired, the first generation unit 104 generates the plurality of tomographic images from the plurality of projection images based on the virtual projection position P4, and then generates the low-energy composite two-dimensional image LEs from the plurality of tomographic images.

Further, the first generation unit 104 may generate the low-energy composite two-dimensional image LEs from the plurality of tomographic images based on the projection path from the virtual projection position P4. That is, the first generation unit 104 generates the plurality of tomographic images from the plurality of projection images without correcting the magnification ratio, and projects the plurality of tomographic images from the virtual projection position P4 to generate the low-energy composite two-dimensional image LEs.

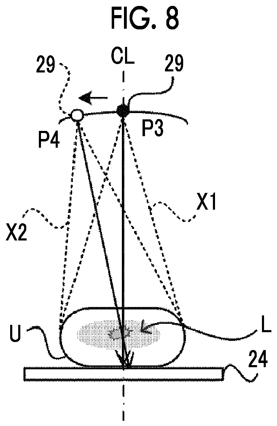

Fig. 8 is a diagram showing the magnification ratio. The first generation unit 104 may generate the plurality of tomographic images by correcting the magnification ratio using the virtual projection position P4 as a center for each of the plurality of projection images, and may generate the low-energy composite two-dimensional image LEs by performing a parallel projection on the plurality of generated tomographic images. In the example of Fig. 8, the irradiation position P3 of the radiation source 29 is corrected to the corresponding virtual projection position P4, but a magnification ratio X1 in a case in which the cone beam-shaped radiation is emitted using the irradiation position P3 as a center and a magnification ratio X2 in a case in which the cone beam-shaped radiation is emitted using the virtual projection position P4 as a center are different. Therefore, the plurality of tomographic images are generated by correcting the magnification ratio X1 to the magnification ratio X2 for each of the plurality of projection images.

Next, a case will be described in which the tomographic image is acquired instead of the projection image and the low-energy composite two-dimensional image LEs is generated using the virtual projection position P4 as an example. The first acquisition unit 101 acquires the plurality of tomographic images. In a case in which the plurality of tomographic images are acquired, the first generation unit 104 generates the low-energy composite two-dimensional image LEs by combining the plurality of tomographic images based on the virtual projection position P4.

In addition, the first acquisition unit 101 may acquire the plurality of tomographic images of which the magnification ratios are corrected using the virtual projection position P4 as a center. In such a case, the first generation unit 104 generates the low-energy composite two-dimensional image LEs by performing a parallel projection on the plurality of acquired tomographic images. That is, the magnification ratio may be corrected at the virtual projection position P4 calculated from the tomographic image itself from the high-energy normal two-dimensional image HE, and the low-energy composite two-dimensional image LEs may be generated from the tomographic image of which the magnification ratio is corrected.

In addition, in a case in which the magnification ratios of the plurality of acquired tomographic images are not corrected or the centers of the correction of the magnification ratios are at different positions, the first generation unit 104 may correct the magnification ratios of the plurality of tomographic images at the virtual projection position P4 and perform a parallel projection on the plurality of corrected tomographic images to generate the low-energy composite two-dimensional image LEs.

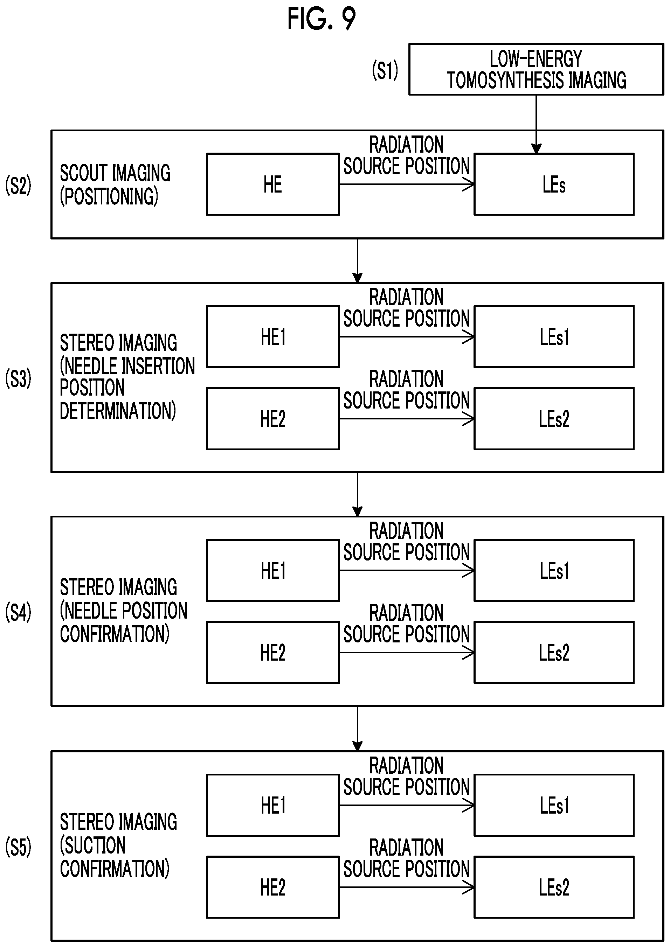

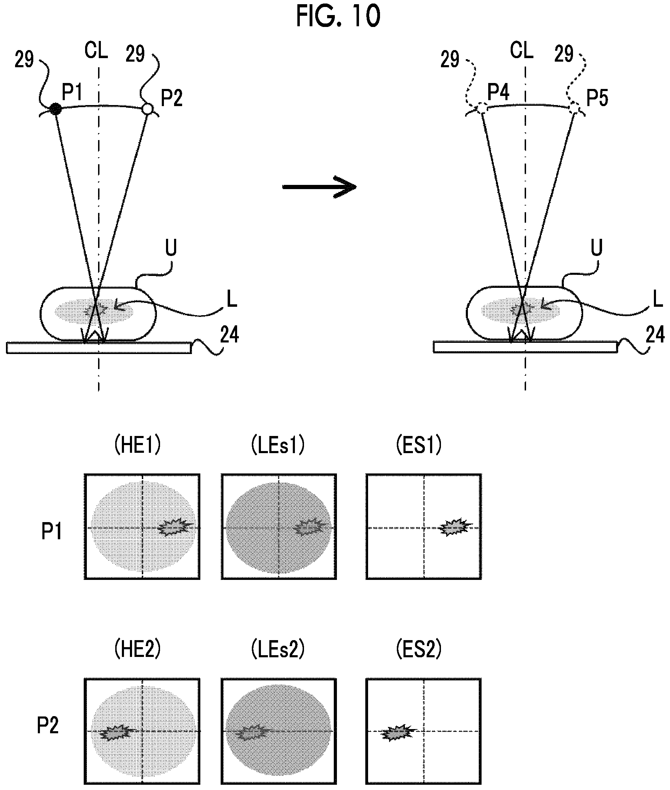

Next, the CEDM biopsy according to the first embodiment will be described in detail with reference to Figs. 9 and 10.

Fig. 9 is a diagram showing a processing flow of the CEDM biopsy according to the first embodiment. In addition, Fig. 10 is a diagram showing the CEDM biopsy according to the first embodiment.

In (S1) of Fig. 9, the breast U is irradiated with radiation having low energy to perform the tomosynthesis imaging, and the series of the plurality of projection images or the plurality of tomographic images are acquired.

In (S2), scout imaging (positioning) for performing positioning is performed. During the scout imaging, the high-energy normal two-dimensional image HE is captured at the irradiation position for the scout imaging, and the corresponding virtual projection position during the tomosynthesis imaging is calculated from the irradiation position of the high-energy normal two-dimensional image HE. Then, the low-energy composite two-dimensional image LEs is generated from the plurality of projection images or the plurality of tomographic images obtained by the tomosynthesis imaging, based on the calculated virtual projection position. It should be noted that the irradiation position for the scout imaging may be, for example, the irradiation position P1 or the irradiation position P2 shown in Fig. 10, or may be an irradiation position other than the irradiation positions P1 and P2.

In (S3), stereo imaging (needle insertion position determination) for determining a position of a needle to be inserted into the breast U is performed. During the stereo imaging in this case, for example, as shown in Fig. 10, the high-energy normal two-dimensional image HE1 is captured at the irradiation position P1 of the radiation source 29, and the high-energy normal two-dimensional image HE2 is captured at the irradiation position P2 of the radiation source 29 by changing the angle. Then, the virtual projection position P4 corresponding to the irradiation position P1 of the high-energy normal two-dimensional image HE1 during the tomosynthesis imaging is calculated, and the low-energy composite two-dimensional image LEs1 is generated from the plurality of projection images or the plurality of tomographic images based on the calculated virtual projection position P4. Similarly, the virtual projection position P5 corresponding to the irradiation position P2 of the high-energy normal two-dimensional image HE2 during the tomosynthesis imaging is calculated, and the low-energy composite two-dimensional image LEs2 is generated from the plurality of projection images or the plurality of tomographic images based on the calculated virtual projection position P5. Then, as shown in Fig. 10, the difference image ES1 between the high-energy normal two-dimensional image HE1 and the low-energy composite two-dimensional image LEs1 is generated, and the difference image ES2 between the high-energy normal two-dimensional image HE2 and the low-energy composite two-dimensional image LEs2 is generated.

In (S4), stereo imaging (needle position confirmation) for confirming a position after the needle is inserted into the breast U is performed. During the stereo imaging in this case, as in the above-described stereo imaging (S3), the high-energy normal two-dimensional images HE1 and HE2 are captured at the irradiation positions P1 and P2, and the low-energy composite two-dimensional images LEs1 and LEs2 are generated from the plurality of projection images or the plurality of tomographic images obtained by the tomosynthesis imaging based on the calculated virtual projection positions P4 and P5. Then, the difference image ES1 between the high-energy normal two-dimensional image HE1 and the low-energy composite two-dimensional image LEs1 is generated, and the difference image ES2 between the high-energy normal two-dimensional image HE2 and the low-energy composite two-dimensional image LEs2 is generated.

In (S5), stereo imaging (suction confirmation) for confirming a state after suctioning a tissue from a hole of the needle is performed. During the stereo imaging in this case, as in the above-described stereo imaging (S3), the high-energy normal two-dimensional images HE1 and HE2 are captured at the irradiation positions P1 and P2, and the low-energy composite two-dimensional images LEs1 and LEs2 are generated from the plurality of projection images or the plurality of tomographic images obtained by the tomosynthesis imaging based on the calculated virtual projection positions P4 and P5. Then, the difference image ES1 between the high-energy normal two-dimensional image HE1 and the low-energy composite two-dimensional image LEs1 is generated, and the difference image ES2 between the high-energy normal two-dimensional image HE2 and the low-energy composite two-dimensional image LEs2 is generated.

Here, the high-energy normal two-dimensional image HE includes a plurality of two-dimensional images captured while changing the irradiation position of the radiation source 29. In the examples of Figs. 9 and 10, the high-energy normal two-dimensional image HE1 is captured at the irradiation position P1, and the high-energy normal two-dimensional image HE2 is captured at the irradiation position P2 by changing the angle.

In addition, in a case in which the movement of the breast U is detected during the tomosynthesis imaging, the first acquisition unit 101 may perform control of performing the tomosynthesis imaging again. Specifically, for example, a message for prompting the user to perform the tomosynthesis imaging again may be displayed. Alternatively, an instruction signal for instructing the mammography apparatus 10 to perform the tomosynthesis imaging again may be transmitted.

In addition, in a case in which the movement of the breast U is detected during the tomosynthesis imaging, the first generation unit 104 may correct the movement of the breast U to generate the low-energy composite two-dimensional image LEs. Specifically, for example, an amount of change in the position of the breast U may be detected, and the movement of the breast U may be corrected based on the detected amount of change.

In addition, the first acquisition unit 101 may detect the movement of the breast U by performing threshold value processing on the difference between the high-energy normal two-dimensional image HE and the low-energy composite two-dimensional image LE. That is, in a case in which the breast U moves during the tomosynthesis imaging, the position of the breast U (that is, the lesion L) in the low-energy composite two-dimensional image LE is shifted. Therefore, it is possible to determine that the breast U has moved in a case in which the difference between the high-energy normal two-dimensional image HE and the low-energy composite two-dimensional image LE is equal to or greater than a threshold value. It should be noted that the movement of the breast U may be detected using, for example, a sensor, a camera (not shown), or the like.

In addition, in a case in which an instruction from the user is received after the tomosynthesis imaging, the first acquisition unit 101 may perform control of performing the tomosynthesis imaging again. Specifically, for example, after the tomosynthesis imaging, in a case in which the user checks the low-energy composite two-dimensional image LE and determines that it is preferable to perform the tomosynthesis imaging again, a screen for receiving an instruction to perform the tomosynthesis imaging again may be displayed.

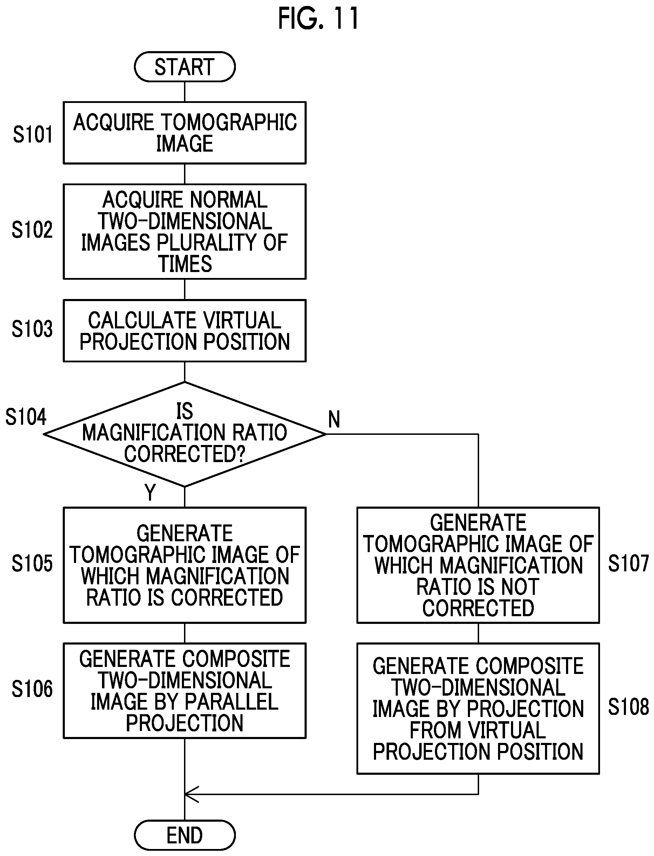

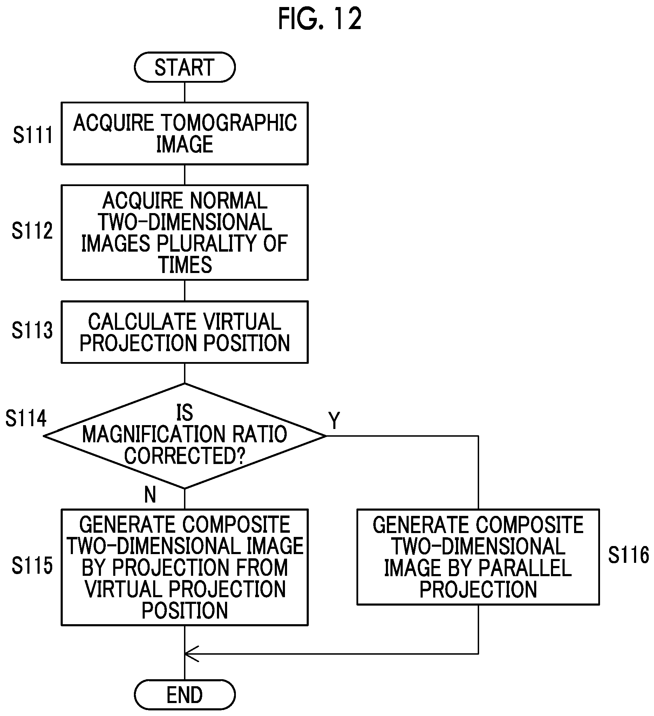

Next, an operation of the image processing apparatus 16 according to the first embodiment will be described with reference to Figs. 11 and 12.

Fig. 11 is a flowchart showing an example of a flow of the processing by the image processing program 62A according to the first embodiment. In Fig. 11, a case will be described in which the projection image is acquired without acquiring the tomographic image.

First, in a case in which the image processing apparatus 16 receives an instruction to start the image processing, the CPU 60A reads out the image processing program 62A and executes the image processing program 62A.

In step S101 of Fig. 11, the CPU 60A acquires the series of the plurality of projection images by the tomosynthesis imaging, as an example, as shown in Fig. 9. The series of the plurality of projection images are images obtained by performing the tomosynthesis imaging by irradiating the breast U with radiation having low energy.

In step S102, the CPU 60A acquires, for example, the plurality of high-energy normal two-dimensional images HE obtained by irradiating the breast U with radiation having high energy a plurality of times as shown in Fig. 9. It should be noted that, in the example of Fig. 9, the high-energy normal two-dimensional images HE for four times of "positioning", "needle insertion position determination", "needle position confirmation", and "suction confirmation" are acquired. In addition, since the stereo imaging is performed in the "needle insertion position determination", the "needle position confirmation", and the "suction confirmation", the high-energy normal two-dimensional image HE is acquired for each of the stereo imaging.

In step S103, the CPU 60A calculates, for example, as shown in Figs. 9 and 10, the virtual projection positions P4 and P5, which are positions at which the breast U is virtually projected during the tomosynthesis imaging, from the irradiation positions P1 and P2 of the radiation source 29 in a case in which the high-energy normal two-dimensional image HE is captured, for each of the plurality of high-energy normal two-dimensional images HE.

In step S104, the CPU 60A determines whether or not to perform the correction of the magnification ratio using the virtual projection positions P4 and P5 as a center for each of the series of the plurality of projection images. In a case in which it is determined to perform the correction of the magnification ratio (in a case in which an affirmative determination is made), the processing proceeds to step S105, and in a case in which it is determined not to perform the correction of the magnification ratio (in a case in which a negative determination is made), the processing proceeds to step S107.

In step S105, the CPU 60A generates the plurality of tomographic images by correcting the magnification ratio using the virtual projection positions P4 and P5 as a center for each of the series of the plurality of projection images.

In step S106, the CPU 60A generates the low-energy composite two-dimensional image LEs by performing a parallel projection on the plurality of generated tomographic images, and completes the series of processing by the image processing program 62A.

On the other hand, in step S107, the CPU 60A generates the plurality of tomographic images for each of the series of plurality of projection images without correcting the magnification ratio.

In step S108, the CPU 60A generates the low-energy composite two-dimensional image LEs by projecting the plurality of generated tomographic images from the virtual projection positions P4 and P5, and completes the series of processing by the image processing program 62A.

Fig. 12 is a flowchart showing another example of the flow of the processing by the image processing program 62A according to the first embodiment. A case in which the tomographic image is acquired instead of the projection image will be described with reference to Fig. 12.

First, in a case in which the image processing apparatus 16 receives an instruction to start the image processing, the CPU 60A reads out the image processing program 62A and executes the image processing program 62A.

In step S111 of Fig. 12, the CPU 60A acquires the plurality of tomographic images by the tomosynthesis imaging. The plurality of tomographic images are images obtained by performing the tomosynthesis imaging by irradiating the breast U with radiation having low energy.

In step S112, the CPU 60A acquires, for example, the plurality of high-energy normal two-dimensional images HE obtained by irradiating the breast U with radiation having high energy a plurality of times as shown in Fig. 9. It should be noted that, in the example of Fig. 9, the high-energy normal two-dimensional images HE for four times of "positioning", "needle insertion position determination", "needle position confirmation", and "suction confirmation" are acquired. In addition, since the stereo imaging is performed in the "needle insertion position determination", the "needle position confirmation", and the "suction confirmation", the high-energy normal two-dimensional image HE is acquired for each of the stereo imaging.

In step S113, the CPU 60A calculates, for example, as shown in Figs. 9 and 10, the virtual projection positions P4 and P5, which are positions at which the breast U is virtually projected during the tomosynthesis imaging, from the irradiation positions P1 and P2 of the radiation source 29 in a case in which the high-energy normal two-dimensional image HE is captured, for each of the plurality of high-energy normal two-dimensional images HE.

In step S114, the CPU 60A determines whether or not the correction of the magnification ratio using the virtual projection position as a center is performed on the plurality of acquired tomographic images. In a case in which it is determined that the correction of the magnification ratio is not performed (in a case in which a negative determination is made), the processing proceeds to step S115, and in a case in which it is determined that the correction of the magnification ratio is performed (in a case in which an affirmative determination is made), the processing proceeds to step S116.

In step S115, the CPU 60A generates the low-energy composite two-dimensional image LEs by projecting the plurality of acquired tomographic images from the calculated virtual projection positions P4 and P5, and completes the series of processing by the image processing program 62A.

On the other hand, in step S116, the CPU 60A generates the low-energy composite two-dimensional image LEs by performing a parallel projection on the plurality of acquired tomographic images, and completes the series of processing by the image processing program 62A.

It should be noted that, in a case in which the correction of the magnification ratio is not performed using the virtual projection positions P4 and P5 as a center for the plurality of tomographic images acquired in step S111 or the centers of the correction of the magnification ratios are at different positions, the magnification ratios of the plurality of tomographic images may be corrected by the virtual projection positions P4 and P5 calculated in step S113, and the low-energy composite two-dimensional image LEs may be generated by the parallel projection.

As described above, according to the present embodiment, during the CEDM biopsy, since the tomosynthesis imaging with low energy with a large exposure dose can be completed only once, the exposure dose can be reduced as compared with the CEDM biopsy of the comparative example. It should be noted that, during the tomosynthesis imaging, the imaging is continuously performed a plurality of times, but the exposure dose in this case is smaller than the exposure dose in a case in which the low-energy normal imaging is performed a plurality of times.

In addition, since the tomosynthesis imaging with low energy can be completed only once, it is not necessary to perform the set imaging for the high-energy normal two-dimensional image and the low-energy normal two-dimensional image, and the imaging time can be shortened.

### Second Embodiment

In the first embodiment, an aspect has been described in which the tomosynthesis imaging is used for imaging with low energy. In the second embodiment, an aspect will be described in which the tomosynthesis imaging is used for imaging with high energy.

First, a functional configuration of an image processing apparatus 16A according to the second embodiment will be described with reference to Fig. 6. In the second embodiment, the first energy is high energy, and the second energy is low energy.

The first acquisition unit 101 acquires the series of the plurality of projection images or the plurality of tomographic images. The series of the plurality of projection images or the plurality of tomographic images are images obtained by performing the tomosynthesis imaging by irradiating the breast U with radiation having high energy.

The second acquisition unit 102 acquires the plurality of low-energy normal two-dimensional images LE obtained by irradiating the breast U with radiation having low energy a plurality of times.

The calculation unit 103 calculates the virtual projection position, which is a position at which the breast U is virtually projected during the tomosynthesis imaging, from the irradiation position of the radiation source 29 in a case in which the low-energy normal two-dimensional image LE is captured, for each of the plurality of low-energy normal two-dimensional images LE.

The first generation unit 104 generates the high-energy composite two-dimensional image from the plurality of projection images or the plurality of tomographic images based on the virtual projection position calculated for each of the plurality of low-energy normal two-dimensional images LE. Hereinafter, the generated high-energy composite two-dimensional image will be referred to as a high-energy composite two-dimensional image HEs in order to distinguish the high-energy composite two-dimensional image from the high-energy normal two-dimensional image HE.

The second generation unit 105 generates the difference image ES between each of the plurality of low-energy normal two-dimensional images LE and each of the high-energy composite two-dimensional images HEs generated for each of the plurality of low-energy normal two-dimensional images LE.

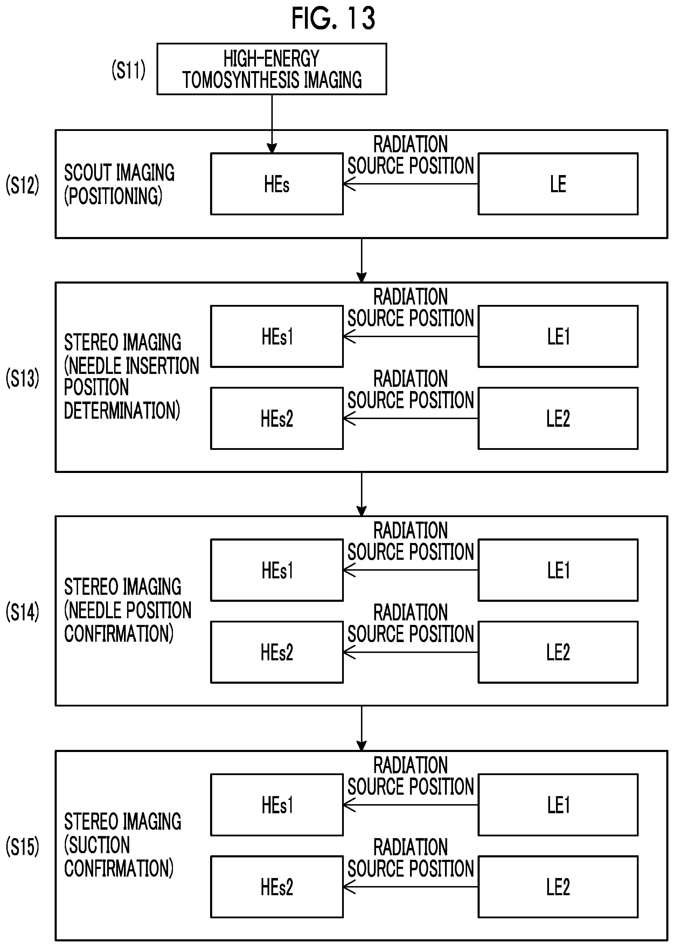

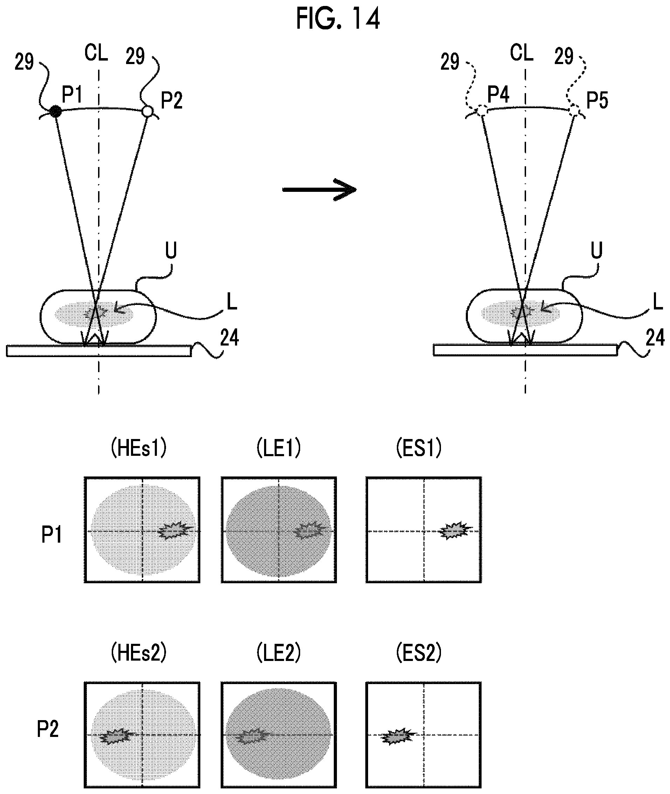

Fig. 13 is a diagram showing a processing flow of a CEDM biopsy according to the second embodiment. Further, Fig. 14 is a diagram showing the CEDM biopsy according to the second embodiment.

In (S11) of Fig. 13, the breast U is irradiated with radiation having high energy to perform the tomosynthesis imaging, and the series of the plurality of projection images or the plurality of tomographic images are acquired.

In (S12), scout imaging (positioning) for performing positioning is performed. During the scout imaging, the low-energy normal two-dimensional image LE is captured at the irradiation position for the scout imaging, and the corresponding virtual projection position during the tomosynthesis imaging is calculated from the irradiation position of the low-energy normal two-dimensional image LE. Then, the high-energy composite two-dimensional image HEs is generated from the plurality of projection images or the plurality of tomographic images obtained by the tomosynthesis imaging, based on the calculated virtual projection position. It should be noted that the irradiation position for the scout imaging may be, for example, the irradiation position P1 or the irradiation position P2 shown in Fig. 14, or may be an irradiation position other than the irradiation positions P1 and P2.

In (S13), stereo imaging (needle insertion position determination) for determining a position of a needle to be inserted into the breast U is performed. During the stereo imaging in this case, for example, as shown in Fig. 14, the low-energy normal two-dimensional image LE1 is captured at the irradiation position P1 of the radiation source 29, and the low-energy normal two-dimensional image LE2 is captured at the irradiation position P2 of the radiation source 29 by changing the angle. Then, the virtual projection position P4 corresponding to the irradiation position P1 of the low-energy normal two-dimensional image LE1 during the tomosynthesis imaging is calculated, and the high-energy composite two-dimensional image HEs1 is generated from the plurality of projection images or the plurality of tomographic images based on the calculated virtual projection position P4. Similarly, the virtual projection position P5 corresponding to the irradiation position P2 of the low-energy normal two-dimensional image LE2 during the tomosynthesis imaging is calculated, and the high-energy composite two-dimensional image HEs2 is generated from the plurality of projection images or the plurality of tomographic images based on the calculated virtual projection position P5. Then, as shown in Fig. 14, the difference image ES1 between the low-energy normal two-dimensional image LE1 and the high-energy composite two-dimensional image HEs1 is generated, and the difference image ES2 between the low-energy normal two-dimensional image LE2 and the high-energy composite two-dimensional image HEs2 is generated.

In (S14), stereo imaging (needle position confirmation) for confirming a position after the needle is inserted into the breast U is performed. During the stereo imaging in this case, as in the above-described stereo imaging (S13), the low-energy normal two-dimensional images LE1 and LE2 are captured at the irradiation positions P1 and P2, and the high-energy composite two-dimensional images HEs1 and HEs2 are generated from the plurality of projection images or the plurality of tomographic images obtained by the tomosynthesis imaging based on the calculated virtual projection positions P4 and P5. Then, the difference image ES1 between the low-energy normal two-dimensional image LE1 and the high-energy composite two-dimensional image HEs1 is generated, and the difference image ES2 between the low-energy normal two-dimensional image LE2 and the high-energy composite two-dimensional image HEs2 is generated.

In (S15), stereo imaging (suction confirmation) for confirming a state after suctioning a tissue from a hole of the needle is performed. During the stereo imaging in this case, as in the above-described stereo imaging (S13), the low-energy normal two-dimensional images LE1 and LE2 are captured at the irradiation positions P1 and P2, and the high-energy composite two-dimensional images HEs1 and HEs2 are generated from the plurality of projection images or the plurality of tomographic images obtained by the tomosynthesis imaging based on the calculated virtual projection positions P4 and P5. Then, the difference image ES1 between the low-energy normal two-dimensional image LE1 and the high-energy composite two-dimensional image HEs1 is generated, and the difference image ES2 between the low-energy normal two-dimensional image LE2 and the high-energy composite two-dimensional image HEs2 is generated.

As described above, according to the present embodiment, during the CEDM biopsy, since the tomosynthesis imaging with high energy can be completed only once, the exposure dose can be reduced as compared with the CEDM biopsy of the comparative example. It should be noted that, during the tomosynthesis imaging, the imaging is continuously performed a plurality of times, but the exposure dose in this case is smaller than the exposure dose in a case in which the high-energy normal imaging is performed a plurality of times.

In addition, since the tomosynthesis imaging with high energy can be completed only once, it is not necessary to perform the set imaging for the high-energy normal two-dimensional image and the low-energy normal two-dimensional image, and the imaging time can be shortened.

Although one form of the image processing apparatus 16 has been described above using the embodiment, the disclosed form of the image processing apparatus 16 is merely an example, and the form of the image processing apparatus 16 is not limited to the range described in the embodiment. Various modifications and improvements can be added to the embodiment without departing from the scope of the present disclosure, and the technical scope of the present disclosure also includes the embodiment to which the modifications or improvements are added.

In the above-described embodiment, as an example, a form has been described in which the control processing of the image processing apparatus 16 is implemented by software processing. However, the control processing of the image processing apparatus 16 may be implemented by hardware. In such a case, the processing speed is increased as compared with a case in which the processing is implemented by software processing.

In the above-described embodiment, the processor refers to a processor in a broad sense, and examples of the processor include a general-purpose processor (for example, the CPU), and a dedicated processor (for example, a graphics processing unit (GPU), an application specific integrated circuit (ASIC), a field programmable gate array (FPGA), a programmable logic device, or the like).

In addition, the operation of the processor in the embodiment described above may be performed not only by one processor but also by cooperation of a plurality of processors existing at physically separated positions. In addition, the order of the operations of the processor is not limited to only the order described in each of the embodiments described above, and may be changed as appropriate.

In the above-described embodiment, an example has been described in which the image processing program 62A is stored in the storage unit 62. However, a storage destination of the image processing program 62A is not limited to the storage unit 62. The image processing program 62A according to the present disclosure can also be provided in a form stored in a computer-readable storage medium. In addition, a form of a computer program product including the image processing program 62A may be adopted. The present disclosure is also applicable to a program and a program product.

For example, the image processing program 62A may be provided in a form of being stored in an optical disk, such as a CD-ROM, a DVD-ROM, and a Blu-ray disc. In addition, the image processing program 62A may be provided in a form of being stored in a portable semiconductor memory, such as a universal serial bus (USB) memory and a memory card. These CD-ROM, DVD-ROM, Blu-ray disc, USB, and memory card are examples of a non-transitory storage medium.

In regard to the embodiment described above, the supplementary notes will be further disclosed as follows.

### Supplementary Note 1

An image processing apparatus comprising: a processor, in which the processor is configured to: acquire a series of a plurality of projection images or a plurality of tomographic images obtained by performing tomosynthesis imaging by irradiating a breast with radiation having a first energy; acquire a plurality of normal two-dimensional images captured by irradiating the breast with radiation having a second energy, which is higher than the first energy, a plurality of times; calculate, for each of the plurality of normal two-dimensional images, a virtual projection position, which is a position at which the breast is virtually projected during the tomosynthesis imaging, from a position of a radiation source in a case in which the normal two-dimensional image is captured; generate a composite two-dimensional image from the plurality of projection images or the plurality of tomographic images based on the virtual projection position calculated for each of the plurality of normal two-dimensional images; and generate a difference image between each of the plurality of normal two-dimensional images and each of the composite two-dimensional images generated for each of the plurality of normal two-dimensional images.

### Supplementary Note 2

The image processing apparatus according to supplementary note 1, in which the processor is configured to, in a case in which the plurality of projection images are acquired, generate the plurality of tomographic images from the plurality of projection images based on the virtual projection position and then generate the composite two-dimensional image from the plurality of tomographic images.

### Supplementary Note 3

The image processing apparatus according to supplementary note 2, in which the processor is configured to generate the composite two-dimensional image from the plurality of tomographic images based on a projection path from the virtual projection position.

### Supplementary Note 4

The image processing apparatus according to supplementary note 2, in which the processor is configured to: generate the plurality of tomographic images by correcting a magnification ratio using the virtual projection position as a center for each of the plurality of projection images; and generate the composite two-dimensional image by performing a parallel projection on the plurality of generated tomographic images.

### Supplementary Note 5

The image processing apparatus according to supplementary note 1, in which the processor is configured to, in a case in which the plurality of tomographic images are acquired, generate the composite two-dimensional image by combining the plurality of tomographic images based on the virtual projection position.

### Supplementary Note 6

The image processing apparatus according to supplementary note 1, in which the processor is configured to acquire the plurality of tomographic images of which magnification ratios are corrected using the virtual projection position as a center.

### Supplementary Note 7

The image processing apparatus according to supplementary note 1, in which the processor is configured to: acquire the plurality of tomographic images and correct magnification ratios of the plurality of tomographic images at the virtual projection position in a case in which the magnification ratios of the plurality of tomographic images are not corrected or centers of the correction of the magnification ratios are at different positions; and generate the composite two-dimensional image by performing a parallel projection on the plurality of tomographic images subjected to the correction.

### Supplementary Note 8

The image processing apparatus according to any one of supplementary notes 1 to 7, in which the normal two-dimensional image includes a plurality of two-dimensional images captured while changing the position of the radiation source.

### Supplementary Note 9

The image processing apparatus according to any one of supplementary notes 1 to 8, in which the processor is configured to, in a case in which movement of the breast is detected during the tomosynthesis imaging, perform control of performing the tomosynthesis imaging again.

### Supplementary Note 10

The image processing apparatus according to any one of supplementary notes 1 to 8, in which the processor is configured to, in a case in which movement of the breast is detected during the tomosynthesis imaging, generate the composite two-dimensional image by correcting the movement of the breast.

### Supplementary Note 11

The image processing apparatus according to supplementary note 9 or 10, in which the processor is configured to detect the movement of the breast by performing threshold value processing on a difference between the normal two-dimensional image and the composite two-dimensional image.

### Supplementary Note 12

The image processing apparatus according to any one of supplementary notes 1 to 8, in which the processor is configured to, in a case in which an instruction from a user is received after the tomosynthesis imaging, perform control of performing the tomosynthesis imaging again.

### Supplementary Note 13

An image processing apparatus comprising: a processor, in which the processor is configured to: acquire a series of a plurality of projection images or a plurality of tomographic images obtained by performing tomosynthesis imaging by irradiating a breast with radiation having a first energy; acquire a plurality of normal two-dimensional images captured by irradiating the breast with radiation having a second energy lower than the first energy a plurality of times; calculate, for each of the plurality of normal two-dimensional images, a virtual projection position, which is a position at which the breast is virtually projected during the tomosynthesis imaging, from a position of a radiation source in a case in which the normal two-dimensional image is captured; generate a composite two-dimensional image from the plurality of projection images or the plurality of tomographic images based on the virtual projection position calculated for each of the plurality of normal two-dimensional images; and generate a difference image between each of the plurality of normal two-dimensional images and each of the composite two-dimensional images generated for each of the plurality of normal two-dimensional images.

### Supplementary Note 14

An image processing method executed by a computer, the image processing method comprising: acquiring a series of a plurality of projection images or a plurality of tomographic images obtained by performing tomosynthesis imaging by irradiating a breast with radiation having a first energy; acquiring a plurality of normal two-dimensional images captured by irradiating the breast with radiation having a second energy, which is higher than the first energy, a plurality of times; calculating, for each of the plurality of normal two-dimensional images, a virtual projection position, which is a position at which the breast is virtually projected during the tomosynthesis imaging, from a position of a radiation source in a case in which the normal two-dimensional image is captured; generating a composite two-dimensional image from the plurality of projection images or the plurality of tomographic images based on the virtual projection position calculated for each of the plurality of normal two-dimensional images; and generating a difference image between each of the plurality of normal two-dimensional images and each of the composite two-dimensional images generated for each of the plurality of normal two-dimensional images.

### Supplementary Note 15

An image processing program causing a computer to execute a process comprising: acquiring a series of a plurality of projection images or a plurality of tomographic images obtained by performing tomosynthesis imaging by irradiating a breast with radiation having a first energy; acquiring a plurality of normal two-dimensional images captured by irradiating the breast with radiation having a second energy, which is higher than the first energy, a plurality of times; calculating, for each of the plurality of normal two-dimensional images, a virtual projection position, which is a position at which the breast is virtually projected during the tomosynthesis imaging, from a position of a radiation source in a case in which the normal two-dimensional image is captured; generating a composite two-dimensional image from the plurality of projection images or the plurality of tomographic images based on the virtual projection position calculated for each of the plurality of normal two-dimensional images; and generating a difference image between each of the plurality of normal two-dimensional images and each of the composite two-dimensional images generated for each of the plurality of normal two-dimensional images.

## Claims

1. An image processing apparatus (16) comprising:
a first acquisition unit (101) being configured to acquire a series of a plurality of projection images or a plurality of tomographic images obtained by performing tomosynthesis imaging by irradiating a breast with radiation having a first energy;
a acquisition unit (102) being configured to acquire a plurality of normal two-dimensional images captured by irradiating the breast with radiation having a second energy, which is higher than the first energy, a plurality of times;
a calculation unit (103) being configured to calculate, for each of the plurality of normal two-dimensional images, a virtual projection position, which is a position at which the breast is virtually projected during the tomosynthesis imaging, from a position of a radiation source in a case in which the normal two-dimensional image is captured;
a first generation unit (104) being configured to generate a composite two-dimensional image from the plurality of projection images or the plurality of tomographic images based on the virtual projection position calculated for each of the plurality of normal two-dimensional images; and
a second generation unit (105) being configured to generate a difference image between each of the plurality of normal two-dimensional images and each of the composite two-dimensional images generated for each of the plurality of normal two-dimensional images.

2. The image processing apparatus (16) according to claim 1,
wherein the first generation unit (104) is configured to, in a case in which the plurality of projection images are acquired, generate the plurality of tomographic images from the plurality of projection images based on the virtual projection position and then generate the composite two-dimensional image from the plurality of tomographic images.

3. The image processing apparatus (16) according to claim 2,
wherein the first generation unit (104) is configured to generate the composite two-dimensional image from the plurality of tomographic images based on a projection path from the virtual projection position.

4. The image processing apparatus (16) according to claim 2,
wherein the first generation unit (104) is configured to:
generate the plurality of tomographic images by correcting a magnification ratio using the virtual projection position as a center for each of the plurality of projection images; and
generate the composite two-dimensional image by performing a parallel projection on the plurality of generated tomographic images.

5. The image processing apparatus (16) according to claim 1,
wherein the first generation unit (104) is configured to, in a case in which the plurality of tomographic images are acquired, generate the composite two-dimensional image by combining the plurality of tomographic images based on the virtual projection position.

6. The image processing apparatus (16) according to claim 1,
wherein the first acquisition unit (101) is configured to acquire the plurality of tomographic images of which magnification ratios are corrected using the virtual projection position as a center.

7. The image processing apparatus (16) according to claim 1,
wherein the first generation unit (104) is configured to:
acquire the plurality of tomographic images and correct magnification ratios of the plurality of tomographic images at the virtual projection position in a case in which the magnification ratios of the plurality of tomographic images are not corrected or centers of the correction of the magnification ratios are at different positions; and
generate the composite two-dimensional image by performing a parallel projection on the plurality of tomographic images subjected to the correction.

8. The image processing apparatus (16) according to any one of claims 1 to 7,
wherein the normal two-dimensional image includes a plurality of two-dimensional images captured while changing the position of the radiation source.

9. The image processing apparatus (16) according to any one of claims 1 to 8,
wherein the first acquisition unit (101) is configured to, in a case in which movement of the breast is detected during the tomosynthesis imaging, perform control of performing the tomosynthesis imaging again.

10. The image processing apparatus (16) according to any one of claims 1 to 8,
wherein the first generation unit (104) is configured to, in a case in which movement of the breast is detected during the tomosynthesis imaging, generate the composite two-dimensional image by correcting the movement of the breast.

11. The image processing apparatus according to claim 9 or 10,
wherein the first acquisition unit (101) is configured to detect the movement of the breast by performing threshold value processing on a difference between the normal two-dimensional image and the composite two-dimensional image.

12. The image processing apparatus according to any one of claims 1 to 8,
wherein the first acquisition unit (101) is configured to, in a case in which an instruction from a user is received after the tomosynthesis imaging, perform control of performing the tomosynthesis imaging again.

13. An image processing method executed by a computer (16), the image processing method comprising:
acquiring (S101, S111) a series of a plurality of projection images or a plurality of tomographic images obtained by performing tomosynthesis imaging by irradiating a breast with radiation having a first energy;
acquiring (S102, S112) a plurality of normal two-dimensional images captured by irradiating the breast with radiation having a second energy, which is higher than the first energy, a plurality of times;
calculating (S103, S113), for each of the plurality of normal two-dimensional images, a virtual projection position, which is a position at which the breast is virtually projected during the tomosynthesis imaging, from a position of a radiation source in a case in which the normal two-dimensional image is captured;
generating (S104-S108, S114-S116) a composite two-dimensional image from the plurality of projection images or the plurality of tomographic images based on the virtual projection position calculated for each of the plurality of normal two-dimensional images; and
generating a difference image between each of the plurality of normal two-dimensional images and each of the composite two-dimensional images generated for each of the plurality of normal two-dimensional images.

14. An image processing program (62A) causing a computer (16) to execute a process comprising:
acquiring a series of a plurality of projection images or a plurality of tomographic images obtained by performing tomosynthesis imaging by irradiating a breast with radiation having a first energy;
acquiring a plurality of normal two-dimensional images captured by irradiating the breast with radiation having a second energy, which is higher than the first energy, a plurality of times;
calculating, for each of the plurality of normal two-dimensional images, a virtual projection position, which is a position at which the breast is virtually projected during the tomosynthesis imaging, from a position of a radiation source in a case in which the normal two-dimensional image is captured;
generating a composite two-dimensional image from the plurality of projection images or the plurality of tomographic images based on the virtual projection position calculated for each of the plurality of normal two-dimensional images; and
generating a difference image between each of the plurality of normal two-dimensional images and each of the composite two-dimensional images generated for each of the plurality of normal two-dimensional images.
